(19) Europäisches Patentamt

European Patent Office

Office européen des brevets

(11) EP 4 548 959 A1

(12) **EUROPEAN PATENT APPLICATION**

(43) Date of publication:
07.05.2025 Bulletin 2025/19

(21) Application number: 23208090.3

(22) Date of filing: 06.11.2023

(51) International Patent Classification (IPC):
A61N 1/32 (2006.01)          A61N 1/36 (2006.01)

(52) Cooperative Patent Classification (CPC):
A61N 1/323; A61N 1/36034; A61N 1/36171

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: **Foundation for Research on Information
Technologies in Society**
**8004 Zürich (CH)**

(72) Inventor: **The designation of the inventor has not
yet been filed**

(74) Representative: **Schmauder & Partner AG
Patent- & Markenanwälte VSP
Zwängiweg 7
8038 Zürich (CH)**

Remarks:
Claims 16-17 are deemed to be abandoned due to
non-payment of the claims fees (Rule 45(3) EPC).

(54) **APPARATUS FOR ELECTRIC FIELD STIMULATION OF BIOLOGICAL TISSUE AND METHOD
OF USING THE SAME**

(57) An apparatus for electric field stimulation of a
biological tissue region comprises:
a) a plurality (N) of at least two electrode pairs, each
electrode being suited for application on a surface segment of, or implantation into, said biological tissue region;
b) for each electrode pair of said plurality, a respective
electric driving source for generating a respective electric
field between electrodes of said pair.

Each electric driving source is configured to generate
a respective sinusoidally time dependent electric field
having a respective carrier frequency and a respective
amplitude, whereby a resultant time dependent electric
field is formed in a region wherein said respective electric
fields spatially overlap. The apparatus comprises control
means for operating all of said electric driving sources in
such manner that each one of said respective sinusoidally time dependent electric fields has a defined normalized time-dependent phase difference with respect to a
reference phase.

Fig. 1

EP 4 548 959 A1

**Description**

The present invention generally relates to an apparatus and to a method for electric field stimulation of a biological tissue region, particularly in a living human body.

Field of the invention

Electric field stimulation of a human or animal organism is generally understood as a method in which applied, generally time-varying electric fields are used to cause a desired reaction in the organism. The method relies on the possibility for an applied electric field to generate an electrophysiological response in biological tissues, for example in the generation of neural action potentials or the modulation of sub-threshold dynamics. Examples of such excitable cells are neurons, muscle cells and some endocrine cells.

One known method of electric field stimulation, which is called interferential stimulation, relies on generating a desired spatio-temporal electric field distribution in the biological tissue region of interest. Apparatuses and methods implementing interferential stimulation are disclosed, e.g., in WO 2016/057855 A1 (MIT) and WO 2021/044168 A1 (ICI).

WO 2016/057855 A1 (MIT) is based on creating a first electric field between electrodes in a first pair of electrodes, and creating a second electric field between electrodes in a second pair of electrodes, such that, firstly, when the two pairs of electrodes are electrically connected to a brain, the first and second electric fields constructively and destructively interfere with each other over time to create an amplitude-modulated waveform, and, secondly, the locations of largest envelope amplitude of the amplitude-modulated waveform can be remote from the electrode. Example applications include stimulation of deep brain regions while avoiding activation of overlaying regions.

WO 2021/044168 A1 (ICI) also relies on using two alternating electric fields which are directed through a biological tissue, the two alternating electric fields having, respectively, a first frequency and a second frequency. The two alternating electric fields provide a combined field in the biological tissue. By varying at least one of the two frequencies, the combined field provides a pulsed interferential stimulation signal.

Summary of the invention

It is the principal object of the present invention to overcome the limitations and disadvantages of currently known apparatuses and methods.

Therefore, according to one aspect, there is provided an apparatus for electric field stimulation of a biological tissue region, the apparatus comprising:

a) a plurality N of at least two electrode pairs, each electrode being formed for application on a surface segment of, or implanted into, said biological tissue region, and
b) for each electrode pair, a respective electric driving source for generating a respective electric field between electrodes of said pair.

In the present context, the term "biological issue region" shall be understood as a region of biological tissue, including, without limitation, a region of biological tissue of an organism, particularly of a living organism, wherein the organism can be an animal or a human, but also including a region of biological tissue constituting a sample in an in-vitro setting.

Each electric driving source is configured to generate a respective sinusoidally time dependent electric field having a respective carrier frequency and a respective amplitude, whereby a resultant time dependent electric field is formed in a region wherein respective electric field vectors spatially overlap.

The apparatus comprises control means for operating all of said electric driving sources in such manner that

- all of said respective frequencies are identical to one preselected carrier frequency $f_0$, the carrier frequency $f_0$ being selected from 500 Hz to 1 MHz,
- each one of said respective sinusoidally time dependent electric fields has a defined normalized time-dependent phase difference for one cycle ($\varphi_i(t)$, with i=1 to N) with respect to a reference phase ($\varphi_0 = 0$), wherein

$$\varphi_i(t) = (i - 1) \cdot \varphi(t)$$

- and, if the frequency of the phase modulation is desired to be $f_m$, then the time-dependent phase difference is given by

$$\varphi_i(t) = (i - 1) \cdot \varphi(t) \cdot f_m.$$

According to another aspect, there is provided a method of electric field stimulation of biological tissue in a biological body by means of an apparatus as defined above, the method comprising carrying out at least one stimulation cycle comprising the steps of:

i) for a preselected number N of electrode pairs, determining a phase modulation function φ(t) in a normalized time range t = 0 to 1 corresponding to a chosen normalized envelope pulse shape function p(t) in said normalized time range;

ii) generating respective sinusoidally time dependent electric fields, each having a preselected time-dependent phase difference ($\varphi_i$(t), with i=1 to N) with respect to a reference phase ($\varphi_0 = 0$), wherein:

$$\varphi_i(t) = (i-1) \cdot \varphi(t)$$

whereby the aligned components of resultant electric field vectors in a region wherein the respective electric fields spatially overlap has a temporal envelope shape corresponding to said normalized envelope pulse shape function p(t) in said normalized time range.

In the following, the term "carrier" will be used to compactly refer to each one of the various sinusoidally time dependent electric fields defined above.

The present invention relies on using a time-dependent phase modulation scheme according to which

- the various carriers interacting with each other to form a resultant time dependent electric fields all have the same carrier frequency,
- each carrier is provided with a respective time-dependent phase,
- the time-dependent phases of the various carriers are set up in such a manner as to generate a chosen temporal envelope shape of the resultant overlapping time-dependent electric field vector components.

As will be understood, it is convenient to express the time-dependent phase of each carrier with respect to a common reference phase, which will also determine the - generally time dependent - phase difference between any pair of carriers.

According to the invention, the time-dependent phase difference ($\varphi_i$(t), with i=1 to N) with respect to a reference phase ($\varphi_0$), to obtain an envelope shape repeating at frequency $f_m$ is set up to be

$$\varphi_i(t) = (i-1) \cdot \varphi(t) \cdot f_m$$

Without loss of generality, the reference phase $\varphi_0$ can be set equal to zero.

For the purposes of the present invention, the carrier frequency is selected in the range of 500 Hz to 1 MHz. The lower frequency limit is chosen so as to be sufficiently high to avoid direct stimulation of the biological tissue. The higher frequency limit is chosen for reasons of design and practicality. The above-mentioned frequencies correspond to vacuum wavelengths in the order of 600 km and 300 m, respectively. Accordingly, for a region of interest in a biological body such as a human or animal patient, which will typically not exceed a size of 1 m, the phase of each carrier at a given instant of time is substantially constant across the entire region of interest. This means that the spatial intensity distribution of the resultant time-dependent electric field envelope in the region of interest is determined by the spatially overlapping distributuions of the various carrier field vectors and by possible material inhomogeneities within the biological tissue. The interferential stimulation according to the present invention sets out to provide a desired temporal variation of the resultant electric field summation envelope.

Advantageous embodiments are defined in the dependent claims and in the examples.

According to one embodiment (claim 2), the electric driving sources each comprise a digital synthesizer, with all digital synthesizers running from a common clock so as to be synchronizable to said common reference phase ($\varphi_0$).

According to another embodiment (claim 3), each one of the electric driving sources comprises an electric network that converts an output signal of a digital synthesizer to a corresponding differential electric voltage stimulus or current stimulus of a desired amplitude that excites an associated electrode pair.

According to a further embodiment (claim 4), the possibility to independently excite two different regions of the biological body or to enhance the excitation of a single region by for example having different dominant electric field vector directions, is provided by the apparatus comprising

c) a further plurality (N') of at least two electrode pairs, each electrode being formed for application on a surface segment of, or implanted in, said biological body,

d) for each electrode pair of said further plurality, a respective further electric driving source for generating a respective electric field between electrodes of said pair,

wherein each further electric driving sources are configured to generate respective sinusoidally time dependent further electric field having a respective further carrier frequency and a respective further amplitude, whereby a resultant time dependent further electric fields are formed in a region wherein said further respective electric field vectors spatially overlap.

In this embodiment, the apparatus comprises further control means for operating all of said further electric driving sources in such manner that

- all of said respective further carrier frequencies are identical to one preselected further carrier frequency ($f_0$'), the further carrier frequency ($f_0$') being selected from 500 Hz to 1 MHz,
- each one of said respective further sinusoidally time dependent electric fields has a preselected time-dependent further phase difference ($\varphi_i$'(t), with i=1 to N') with respect to a further reference phase ($\varphi_0$'), wherein

$$\varphi_i{'}(t) = (i-1) \cdot \varphi'(t)$$

- and, if the frequency of the phase modulation is desired to be $f_m$, then the time-dependent further phase difference is given by

$$\varphi_i{'}(t) = (i-1) \cdot \varphi'(t) \cdot f'_m$$

wherein said carrier frequency ($f_0$) and said further carrier frequency ($f_0$') are spaced apart by a frequency difference ($\Delta f$) which is at least 500 Hz. This ensures that the difference in frequency between all carriers is sufficiently high that there is no direct or interferential stimulation of the biological tissue. In this embodiment, a plurality of two or more substantially independent electrode pairs is implemented.

In an advantageous embodiment of the method (claim 6), the modulation function $\varphi$(t) is obtained according to:

$$\varphi(t) = \frac{4}{N} \cdot acos\left(p(t)^{\frac{1}{a(N)}}\right) \cdot sign\left(\frac{d}{dt} acos\left(p(t)^{\frac{1}{a(N)}}\right)\right)$$

wherein a(N) is an envelope correction factor.

In particular (claim 7), when the plurality of electrode pairs is larger than two, the envelope correction factor a(N) is

$$a(N) = 1 + 0.25 \cdot \left(1 - e^{-\frac{N-2}{1.356}}\right)$$

In many embodiments (claim 8), the method steps i) and ii) as defined above are carried out repetitively so as to form a temporal sequence of stimulation cycles. The number of repetitions will generally depend on the specific application.

In many embodiments, the stimulation cycles are identical.

However, according to another embodiment (claim 9), a first normalized envelope pulse shape function p1(t) is chosen in a first normalized time range and a second normalized envelope pulse shape function p2(t) is chosen in a second normalized time range that follows after the first normalized time range, wherein said first and second normalized envelope pulse shapes are selected so as to smoothly switch from said first normalized envelope pulse shape function p1(t) to said second normalized envelope pulse shape function p2(t) synchronously for all electrode pairs of a plurality. In the course of such a temporal sequence, the interferential envelope repetition rate or frequency can be modified, for which purpose phase modulation data is read out of a phase modulation memory block at a controlled rate so as to ensure both carrier and envelope phase continuity when modifying the interferential envelope repetition rate or frequency ($f_m$) (claim 10). For this purpose, one can arrange to have two working data sets for modulation, with switching from one to the other only when the address counter resets to the first data element.

In one class of embodiments using a plurality of two or more substantially independent electrode pairs (claim 11), each plurality of carriers is phase modulated such that the interferential superposition of the respective electric fields has the same envelope shape. In another class of such embodiments (claim 12), each plurality of carriers is phase modulated such that the interferential superposition of the respective electric fields is different for each plurality.

According to one embodiment (claim 15), the method of the invention is used for generating two different sham exposure states where the respective field vectors are mostly aligned in a biological tissue of interest, in which no time varying interferential envelope modulation p(t) is present. In the first sham exposure state, the carrier frequency induced fields in the biological tissue are at a minimum, whereas in the second sham exposure state the carrier frequency induced fields in the biological tissue are at a maximum. This procedure allows modification of neuronal dynamics with modulated envelopes to be compared to HF carrier present or not present at the tissue target.

A further embodiment exploits the fact that many excitable cells, for example neurons, have varying levels of excitability depending on the direction of the electric field vector in the tissue, e.g. neurons being more excitable when aligned with the field vector, the apparatus comprises

c) a plurality N of at least two electrode pairs, each electrode being formed for application on a surface segment of, or implanted in, said biological body,
d) for each electrode pair of said plurality, a respective electric driving source for generating a respective electric field between electrodes of said pair,

Wherein the electrode placements are chosen to obtain essentially orthogonal field vectors in the region of interest and the time-dependent phase difference $\varphi_i(t)$ such that the phase varies between 0 and 360° providing in two orthogonal directions time dependent excitations in antiphase to each other by virtue of the angular variation in excitability of the tissue. In other directions the variation in the magnitude of the envelope is reduced or negligible. The shape of the envelope in the most favorable directions can be modified by varying the rate of change of phase across the envelope period.

According to another embodiment, the time-dependent phase difference ($\varphi_i(t)$, with i=1 to N) used to obtain the required envelope shape

$$\varphi_i(t) = (i - 1) \cdot \varphi(t) \cdot f_m$$

has a frequency $f_m$ that can be modified, for example using a repetitive input trigger pulse, to synchronize the amplitude modulation envelope phase and frequency with the external input trigger which could for example be derived from measured neuronal activity. In this embodiment the rate of reading of the phase data $\varphi_i(t)$ is the quantity modified to achieve the carrier envelope phase lock.

According to a further embodiment, the possibility to independently phase lock the excitation of two different regions of the biological body with respect to two external synchronization pulse streams relating to

c) a plurality (N) of at least two electrode pairs, each electrode being formed for application on a surface segment of, or implanted in, said biological body, with an envelope pulse shape p(t) giving phase modulation data $\varphi_i(t)$
d) and a further plurality (N') of at least two electrode pairs, each electrode being formed for application on a surface segment of, or implanted in, said biological body, operating at a substantially different carrier frequency with an envelope pulse shape p'(t) giving phase modulation data $\varphi'_i(t)$.

Brief description of the drawings

The above mentioned and other features and objects of this invention and the manner of achieving them will become more apparent and this invention itself will be better understood by reference to the following description of various embodiments of this invention taken in conjunction with the accompanying drawings, wherein

Fig. 1    shows a schematic representation of an apparatus according to the invention,

Fig. 2    shows the superposition of two carrier waves with equal amplitude and orientation,

Fig. 3    shows the superposition of three, four and five carrier waves with different frequencies where the carrier frequency spacing is equal and all have equal amplitude,

Fig. 4 shows the relative carrier phase variation in degrees over one envelope period for the case of two carriers with different frequencies producing a sinusoidal amplitude variation,

Fig. 5 shows the phase shift $\varphi(t)$ in degrees producing a sinusoidal amplitude variation between consecutively numbered carrier pairs for the cases of (a) three and (b) four carriers,

Fig. 6 shows the resulting envelope for the superposition of eight equal amplitude carriers (blue dots) using the phase modulation according to the present invention, without envelope correction, versus the desired sinusoidal envelope (solid red),

Fig. 7 shows phase in degrees as a function of amplitude for the second carrier in a two carrier scenario, which can be used to translate a given desired envelope amplitude as a function of time p(t) to the required carrier phase difference as a function of time $\varphi(t)$,

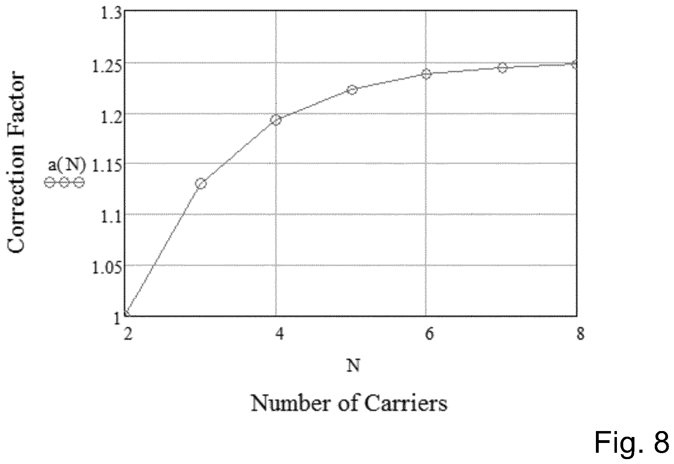

Fig 8 shows the compensation factors for the cases N = 2 to N = 8,

Fig. 9 shows various examples of envelope pulse shape p(t), required phase modulation $\varphi(t)$ in degrees and the resultant superposition at a location where field vectors are aligned and of equal amplitude,

Fig. 10 shows a diagram of polarization ellipses illustrating the range of possible polarizations achievable using polarization modulation for the simplest case in 2D with two orthogonal fields E0 and E1 of equal amplitude for a range of different phase shifts, the two linear polarizations as well as circular polarization being highlighted,

Fig. 11 shows an apparatus that consists of a plurality of sinusoidal sources that can be phase modulated, wherein each source feeds an electric network that converts the output of the source to a differential electric stimulus (voltage or current) of a desired amplitude,

Fig. 12 shows a DDS architecture for seamless transition from one interferential modulation envelope shape to another, and

Fig. 13 shows an architecture for the Mod Clock of Figures 10 and 11 that will allow phase and frequency locking to an external pulse stream, where the division ratio R is the length of the phase modulation data.

## Detailed description of the invention

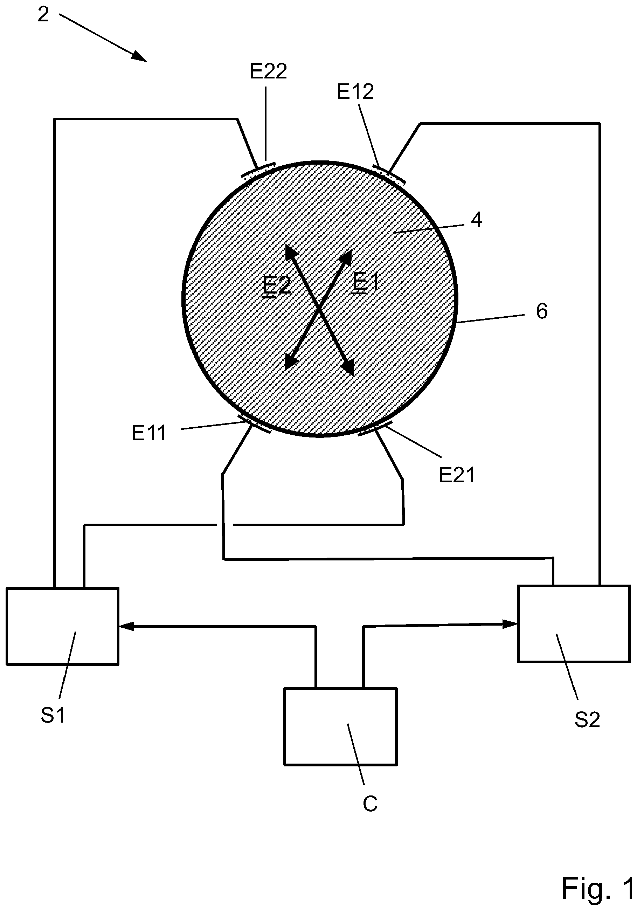

In order to better explain the general principles of the present invention, Fig. 1 shows an overall schematic view of an apparatus 2 for electric field stimulation of biological tissue 4 either in a biological body 6 or in vitro. In the example shown, the apparatus comprises a first electrode pair E11 and E12 and a second electrode pair E21 and E22. Each electrode is applied on a surface segment or implanted in the biological body 6. Each electrode pair is connected to a respective electric driving source S1, S2 for generating a respective electric sinusoidally time dependent field E1 and E2 in the region R between the electrodes of said pair:

$$\underline{E}_i(t,\underline{r}) = \underline{E}_i(\underline{r}) \cdot \sin\left(f_0\, t + \varphi_i(t)\right) \qquad (0)$$

with i =1 or 2 and with $\underline{r}$ being a vector defining position within the overlap region R.

Each one of said time dependent fields has a carrier frequency $f_0$ and a respective amplitude $\underline{E}_i(\underline{r})$ and direction.

A resultant time dependent electric field results from vector summation of the respective time dependent fields generated by the electrode pairs $\underline{E}\,(t,\,\underline{r}) = \underline{E}_1(t,\,\underline{r}) + \underline{E}_2(t,\underline{r})$.

The apparatus further comprises control means C for operating the various electric driving sources S1, S2 in such manner that each one of the respective sinusoidally time dependent electric fields has a time-dependent phase difference ($\varphi_i(t)$, with i=1 to N) with respect to a reference phase ($\varphi_0$), wherein $\varphi_i(t) = (i - 1) \cdot \varphi(t)$.

## Background theory

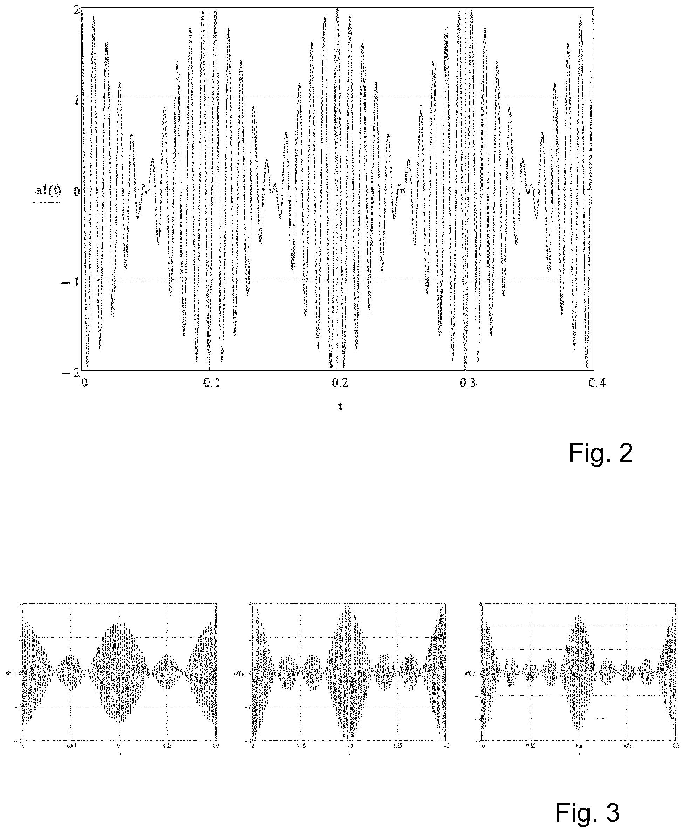

If two carriers of different frequencies are superimposed (summed) the resultant is an amplitude modulated waveform where the amplitude modulation is at $\Delta f$ the difference in frequency between the two carriers. Consider the case where the carriers have the same amplitude (see Fig. 2)

$$a1(t) = \cos\left(\omega_0 \cdot t\right) + \cos\left(\omega_1 \cdot t\right)$$

(1)

The envelope is sinusoidal with phase inversion of the resultant at the zero crossings and the amplitude modulation has a modulation index of 1. If the carriers have different amplitudes the modulation index decreases.

In this simplest case, there is no control over the shape of the envelope and the extension to a number of carriers greater than two using the same paradigm by adding carriers at multiples of $\Delta f$ does not result in similar envelope shapes, but instead adds additional side lobes while narrowing the main signal lobe width (see Fig. 3).

*Phase-Modulation-Approach*

Firstly, it is clear that the maximum envelope amplitude always occurs when all carriers are in phase and the field vectors aligned - for the two-carrier case the minimum envelope amplitude occurs when in antiphase (180°). Furthermore from Fig. 3 for more than two carriers it is clear that the maxima still occurs when all are in phase and that other conditions exist for the minima. Considering the cases in Fig. 3, we can extract for each zero magnitude of the modulation envelope the phases of the carriers with respect to the reference carrier (i=1).

Table 1: Phase shifts in degrees for zero amplitude of the modulation envelope when all carriers have equal amplitude and orientation

| Two sinusoids | Three sinusoids | | Four Sinusoids | | | Five sinusoids | | | |
|---|---|---|---|---|---|---|---|---|---|
| 180 | 120 | -120 | 180 | 90 | -90 | 72 | 144 | -144 | -72 |
| | -120 | 120 | 0 | -180 | 180 | 144 | -72 | 72 | -144 |
| | | | 180 | -90 | 90 | -72 | -144 | 144 | 72 |
| | | | | | | -144 | 72 | -72 | 144 |

We can generalize the phase shift between carriers to achieve cancellation to be 360/N° where N is the number of carriers.

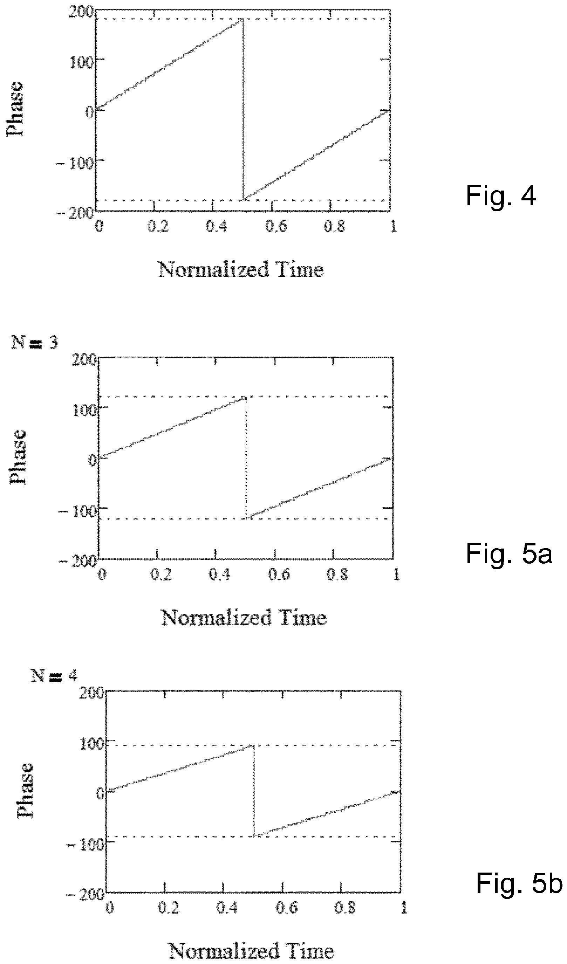

Taking the simplest case of two carriers with different frequencies the resultant envelope is sinusoidal in shape (when the phase inversion is taken into account), with constant frequency difference the relative phase varies linearly over time, see Fig. 4, however, the envelope "pulse" amplitude is not a linear function, see Fig. 2.

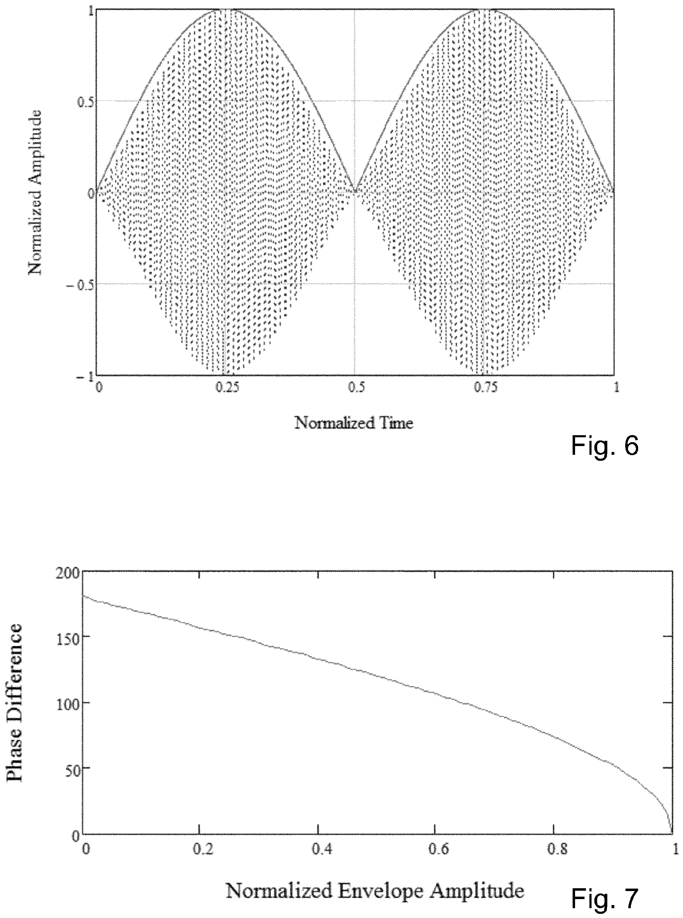

The range of phase variation has to be modified when additional carriers are added to reflect the findings in Table 1, see Fig. 5. All carriers are at the same frequency, the first has no phase modulation, the second has the phase modulation shown in Figure 4 or 5 for the respective number of carriers, then additional carriers have increasing multiples of the phase shift shown in the respective figure. This approach with linear phase difference variation over time results in envelopes approximately the same as that for two carriers of different frequency, note that the required phase shift range decreases inversely with the number of carriers. However, as carriers are added for N>2 the envelope shape departs from the ideal expected if a linear phase increment with time is maintained, see Fig. 6. Hence, a correction factor will need to be applied to achieve exact envelope shapes for the cases where N>2.

Where another envelope shape than that shown in figure 2 is required, the mapping of amplitude to phase for amplitudes normalized between 0 and 1 for two carriers is simply the arc cosine of the amplitude as shown in Fig. 7. For amplitudes between 0 and -1 (the resultant carrier inverted) the phase would be negative.

*Method of the Invention*

Here we describe a method of phase modulation allowing the generation of arbitrary envelope pulse shapes from the superposition of an arbitrary number of carrier signals (N $\geq$ 2) where all the carriers are at the same frequency. The fidelity of the target pulse shape approximation increases with increasing N.

Consider a required pulse p(t) where the amplitude is normalized $0 \leq p(t) \leq 1$ and the pulse has a duration of 1.

The required phase modulation $\varphi(t)$ required to give an envelope shape p(t) for N carriers is given by

$$\varphi(t) = \frac{4}{N} \cdot acos\left(p(t)^{\frac{1}{a(N)}}\right) \cdot sign\left(\frac{d}{dt} acos\left(p(t)^{\frac{1}{a(N)}}\right)\right)$$

(2)

where the constant a(N) is a correction factor to compensate for the effect seen in Fig. 6, where

$$a(N) = 1 + 0.25 \cdot \left(1 - e^{-\frac{N-2}{1.356}}\right)$$

(3)

The values of a(N) can be seen for N in the range 2 to 8 in Fig. 7.

In equation (2), the sign of the differential is included to compensate for the fact that the arc cosine function only results in angles between 0 and 180 degrees.

The resultant assuming aligned field vectors and equal amplitudes of the carriers can be calculated using equation (4), where all phase shifts are multiples of the calculated phase or, alternatively, as per equation (5), where phase shifts have both positive and negative multipliers and are equally spaced around zero shift. Both result in the same envelope.

$$w(N,t) = \sum_{i=0}^{N-1} \cos\left(\omega \cdot t + i \cdot \varphi\left(f_m \cdot t\right)\right)$$

(4)

$$w(N,t) = \sum_{i=0}^{N-1} \cos\left[\omega \cdot t + \left(i - \frac{N}{2}\right) \cdot \varphi\left(f_m \cdot t\right)\right]$$

(5)

*Examples*

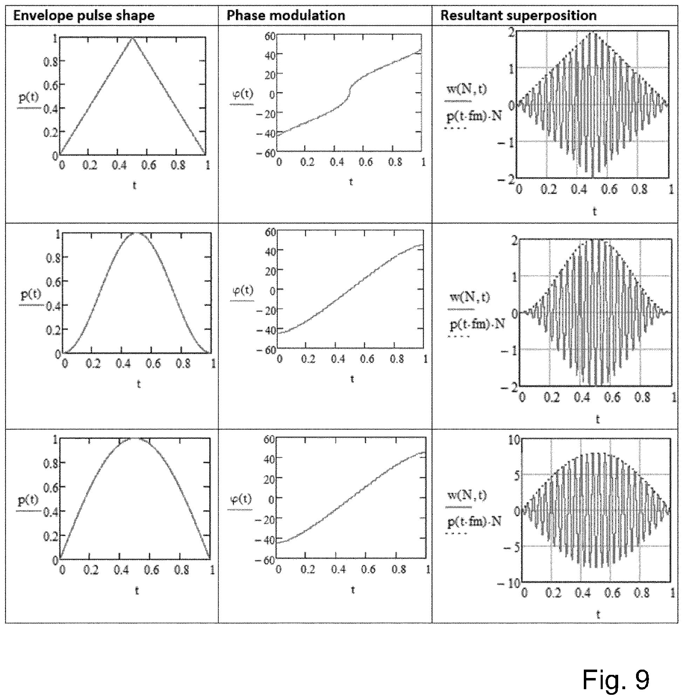

On the left side of Fig. 9, three different examples of normalized envelope amplitude modulation pulse shapes p(t) as a function of normalized time $t \in [0,1)$ are shown, namely a symmetric triangular pulse (upper trace), a Gaussian-type pulse (middle trace) and a sinusoidal type pulse (bottom trace). Also shown in Fig. 9, in the middle column, are the corresponding phase modulation functions $\varphi_i(t)$ and, on the right hand side, the resultant superpositions for the case of N = 8 superposed phase modulated electric fields, which form the interferential modulation of the fast oscillating signals with an envelope substantially matching the desired normalized envelope pulse shape.

*Polarization modulation method*

Here we describe a method of electric field stimulation of biological tissues that exhibit variation in excitability with excitation field angle by means of phase modulation and electrode placement to produce in the target tissue electric fields of time dependent polarization. The placement of each electrode pair is chosen such that they generate respective electric field between the electrodes with a given direction in the target region. The time dependent phase difference between each pair of electrodes in the plurality is chosen such that the superposition of the fields results in a time dependent variation in polarization.

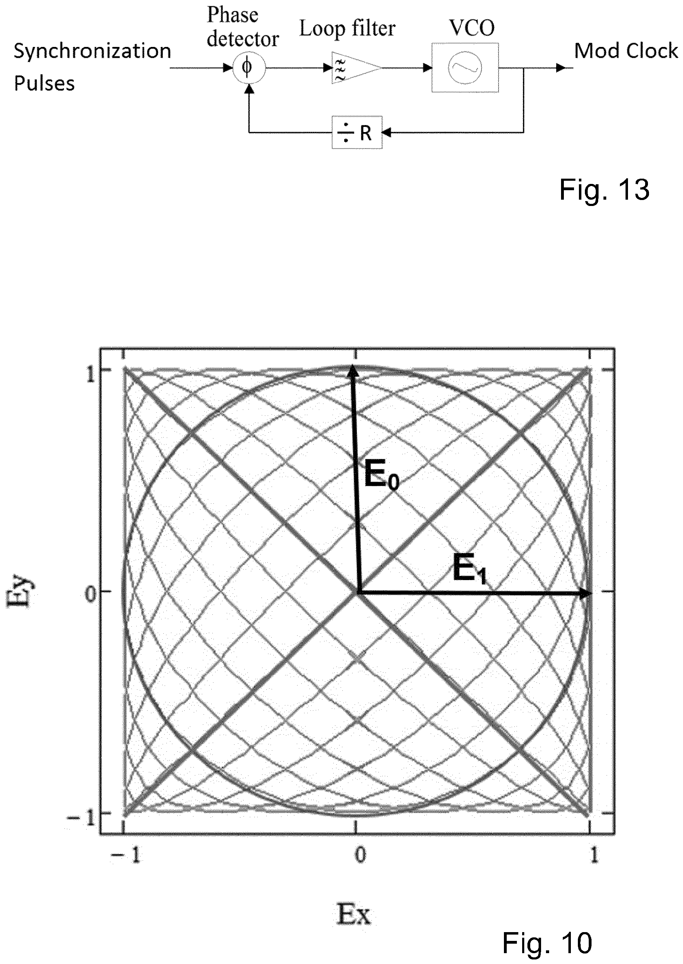

In the simplest form two electrode pairs are placed such that their respective electric fields in the target region are orthogonal to each other. Then, as the phase difference progresses from 0 to 360°, the electric field polarization will vary from linear through elliptical to circular to elliptical and back to linear, whereby the varying alignment of fields within tissues will result in varying levels of excitation depending on the direction of the electric field vector, see Fig. 10. Where the phase difference is time dependent, the tissue will experience a time dependent excitation of the tissue.

The time-dependent phase differences $\varphi_i(t)$ can be chosen such that the rate of change of electric field polarization is modified such that the product of the angular excitability and polarization angle result in a desired excitation as a function of time:

$$\text{Excitation(t)} = \text{Excitability}(\Theta, \Phi) \bullet E(\Theta, \Phi, t)$$

where $\Theta$ and $\Phi$ are the spherical coordinates for vectors

*Apparatus*

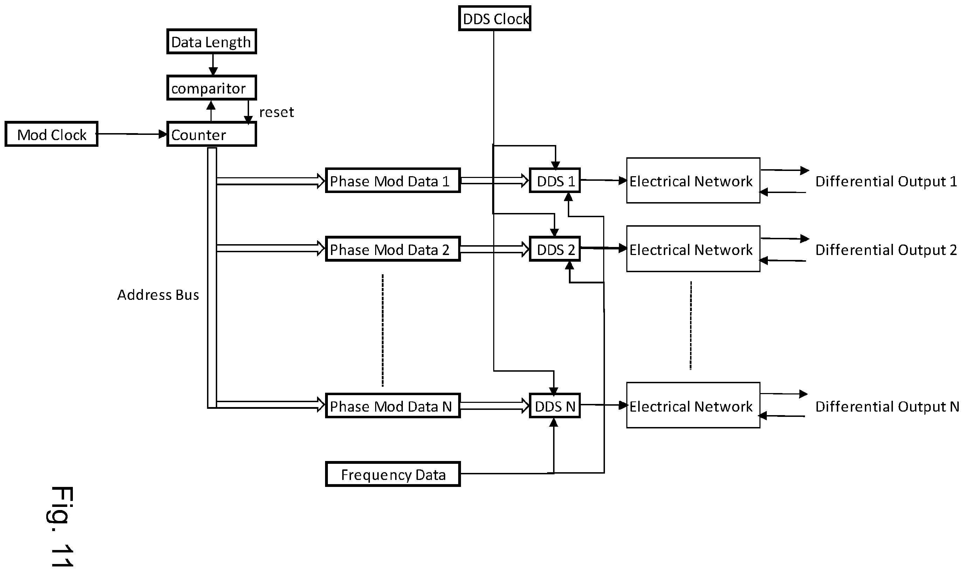

The exemplary apparatus shown in Fig. 11 comprises a plurality of N sinusoidal sources configured as outputs of respective direct digital synthesizers (DDS) that can be phase modulated. All of these source channels run from common digital clock and can be synchronized to have the same initial frequency and phase. A calculated phase modulation can then be applied synchronously to all channels, each block of phase modulation data has the same data length and the appropriate values $\varphi_i(t)$, where i=1 to N. Time t is handled as discrete variable with steps in increments of 1/D, where D is the data length, and the data is for one normalized period of 1. The required modulation envelope period $t_m$ is defined by the modulation clock $f_{data}$ which is set to $D/t_m$ and can be varied at any time to change the envelope frequency while maintaining continuity of both carrier and envelope.

Each phase modulated sinusoidal source feeds an electrical network that converts the output of the source to a differential electrical stimulus (voltage or current) of a desired amplitude that can then be used to excite a pair of electrodes (implanted or in surface contact with the biological body) in order to induce electric fields between the electrodes inside the biological body.

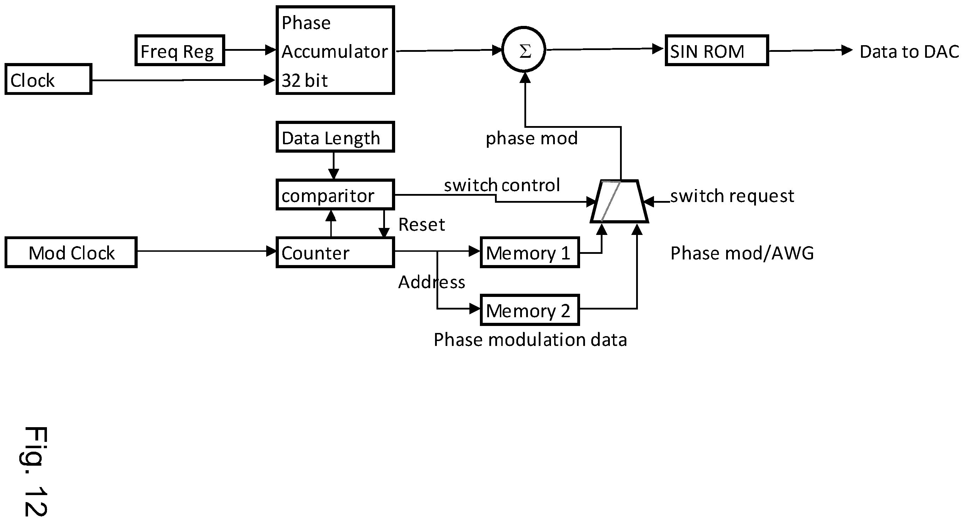

A further refinement, as illustrated in Fig. 12, can introduce a second block of phase modulation data and switched between the two as user requires, providing switching over takes place only when the address counter is resetting to zero, i.e. at the end of a full cycle of data, this will ensure that the transition is controlled. The respective blocks of data can be written at any time providing that block of data is not in active use.

A further refinement, as illustrated in Fig. 13, where the modulation data clock $f_{data}$ that controls the interferential envelope repetition rate or frequency (fm) can be modified in light of an external input trigger pulse train to synchronize the amplitude modulation envelope phase and frequency with for example measured neuronal activity. This can be achieved by means of a phase locked loop which can be implemented in either hardware or digital domains.

**Claims**

1. An apparatus for electric field stimulation of a biological tissue region, the apparatus comprising:

   a) a plurality (N) of at least two electrode pairs, each electrode being suited for application on a surface segment of, or implantation into, said biological tissue region;
   b) for each electrode pair of said plurality, a respective electric driving source for generating a respective electric field between electrodes of said pair,
   wherein each electric driving source is configured to generate a respective sinusoidally time dependent electric field having a respective carrier frequency and a respective amplitude,
   whereby a resultant time dependent electric field is formed in a region wherein said respective electric fields spatially overlap,
   **characterized in that**
   the apparatus comprises control means for operating all of said electric driving sources in such manner that

   - all of said respective frequencies are identical to one preselected carrier frequency ($f_0$), the carrier frequency $f_0$ being selected from 500 Hz to 1 MHz,
   - each one of said respective sinusoidally time dependent electric fields has a defined normalized time-dependent phase difference for one cycle ($\varphi_i(t)$, with i=1 to N) with respect to a reference phase ($\varphi_0 = 0$), wherein

$$\varphi_i(t) = (i - 1) \cdot \varphi(t)$$

   - and, if the frequency of the phase modulation is desired to be $f_m$, then the time-dependent phase difference

is given by

$$\varphi_i(t) = (i - 1) \cdot \varphi(t) \cdot f_m$$

2. The apparatus according to claim 1, wherein said electric driving sources each comprise a digital synthesizer, all of said digital synthesizers running from a common clock so as to be synchronizable to said common reference phase ($\varphi_0$).

3. The apparatus according to claim 2, wherein each one of said electric driving sources comprises an electric network that converts an output signal of a digital synthesizer to a corresponding differential electric voltage stimulus or current stimulus of a desired amplitude that excites an associated electrode pair.

4. The apparatus according to one of claims 1 to 3, comprising

c) a further plurality (N') of at least two electrode pairs, each electrode being formed for application on a surface segment of, or implantation into, said biological tissue region,
d) for each electrode pair of said further plurality, a respective further electric driving source for generating a respective electric field between electrodes of said pair,
wherein each further electric driving source is configured to generate a respective sinusoidally time dependent further electric field having a respective further carrier frequency and a respective further amplitude,
whereby a resultant time dependent further electric field is formed in a region wherein said further respective electric fields spatially overlap,
the apparatus comprising further control means for operating all of said further electric driving sources in such manner that

- all of said respective further carrier frequencies are identical to one preselected further carrier frequency ($f_0'$), the further carrier frequency ($f_0'$) being selected from 500 Hz to 1 MHz,
- each one of said respective further sinusoidally time dependent electric fields has a defined normalized time-dependent further phase difference for one cycle ($\varphi_i'(t)$, with i=1 to N') with respect to a further reference phase ($\varphi_0' = 0$), wherein

$$\varphi_i{}'(t) = (i - 1) \cdot \varphi'(t)$$

- and, if the frequency of the phase modulation is desired to be $f_m$, then the time-dependent further phase difference is given by

$$\varphi_i{}'(t) = (i - 1) \cdot \varphi'(t) \cdot f'_m$$

wherein said carrier frequency ($f_0$) and said further carrier frequency ($f_0'$) are spaced apart by a frequency difference ($\Delta f$) which is at least 500 Hz.

5. A method of electric field stimulation of a biological tissue region by means of an apparatus according to claim 1, the method comprising carrying out at least one stimulation cycle comprising the steps of:

i) for a preselected number (N) of electrode pairs, determining a phase modulation function $\varphi(t)$ in a normalized time range t = 0 to 1 corresponding to a chosen normalized envelope pulse shape function p(t) in said normalized time range;
ii) generating respective sinusoidally time dependent electric fields, each having a preselected time-dependent phase difference ($\varphi_i(t)$, with i=1 to N) with respect to a reference phase ($\varphi_0$), wherein:

$$\varphi_i(t) = (i - 1) \cdot \varphi(t) \cdot f_m$$

whereby the resultant electric field in a region wherein the respective aligned electric field vectors spatially overlap has a temporal envelope shape corresponding to said normalized envelope pulse shape function p(t) in said normalized time range.

6. The method according to claim 5, wherein the modulation function φ(t) is obtained according to:

$$\varphi(t) = \frac{4}{N} \cdot acos\left(p(t)^{\frac{1}{a(N)}}\right) \cdot sign\left(\frac{d}{dt} acos\left(p(t)^{\frac{1}{a(N)}}\right)\right)$$

wherein a(N) is an envelope correction factor.

7. The method according to claim 6, wherein N>2, and wherein the envelope correction factor a(N) is

$$a(N) = 1 + 0.25 \cdot \left(1 - e^{-\frac{N-2}{1.356}}\right)$$

8. The method according to claim 5, wherein steps i) and ii) are carried out repetitively so as to form a temporal sequence of stimulation cycles.

9. The method according to claim 8, wherein a first normalized envelope pulse shape function p1(t) is chosen in a first normalized time range and a second normalized envelope pulse shape function p2(t) is chosen in a second normalized time range that follows after the first normalized time range, wherein said first and second normalized envelope pulse shapes are selected so as to smoothly switch from said first normalized envelope pulse shape function p1(t) to said second normalized envelope pulse shape function p2(t) synchronously for all electrode pairs of a plurality.

10. The method according to claim 9, wherein phase modulation data is read out of a phase modulation memory block at a controlled rate so as to ensure both carrier and envelope phase continuity when modifying the interferential envelope repetition rate or frequency ($f_m$).

11. The method according to claim 5 by means of an apparatus according to claim 4, wherein each plurality of carriers is phase modulated such that the interferential superposition of the respective electric fields has the same envelope shape and frequency.

12. The method according to claim 5 by means of an apparatus according to claim 4, wherein each plurality of carriers is phase modulated such that the interferential superposition of the respective electric fields is different for each plurality.

13. The method according to claim 10, whereby the interferential envelope repetition rate or frequency ($f_m$) can be modified to synchronize the amplitude modulation envelope phase and frequency with an external input trigger pulse train. In this embodiment the rate of reading of the phase data $\varphi_i(t)$ is the quantity modified to achieve the carrier envelope phase lock. -->to be placed into the description

14. The method according to claims 12 and 13, wherein the excitation envelopes of two different regions of the biological tissue region are independently phase locked with respect to two external synchronization pulse streams.

15. The method according to claim 5 for generating two different sham exposure states in a biological tissue of interest, in which no time varying interferential envelope modulation p(t) is present, wherein in the first sham exposure state the carrier frequency induced fields in the biological tissue minimize the total field magnitude and wherein in the second sham exposure state the carrier frequency induced fields in the biological tissue all are in phase.

16. A method of electric field stimulation of biological tissue in a biological tissue region by means of an apparatus according to claim 1, wherein the combination of

   a) the placement of each electrode pair of said plurality is chosen such that the generates respective electric field between each pair of said electrodes with a given direction in the target region
   b) the time dependent phase difference between each pair of electrodes in the plurality

is chosen such that the superposition of the fields results in a time dependent variation in polarization of from linear through elliptical and back to linear whereby appropriate alignment of fields within tissues having varying levels of excitability depending on the direction of the electric field vector experience a time dependent excitation of the tissue.

17. The method according to claim 16 where the time-dependent phase differences $\varphi_i(t)$ are chosen such that the rate change electric field polarization is modified such that the product of the angular excitability and polarization angle result in a desired excitation as a function of time.

2

E22    E12

E2    E1

4

6

E11    E21

S1    C    S2

Fig. 1

EP 4 548 959 A1

Fig. 2

Fig. 3

Fig. 4

N = 3

Fig. 5a

N = 4

Fig. 5b

Fig. 6

Fig. 7

Fig. 8

Fig. 9

18

Fig. 11

Fig. 12

Fig. 13

Fig. 10

Europäisches
Patentamt

European
Patent Office

Office européen
des brevets

# EUROPEAN SEARCH REPORT

Application Number

EP 23 20 8090

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2021/044168 A1 (IMPERIAL COLLEGE INNOVATIONS LTD [GB]) 11 March 2021 (2021-03-11) * the whole document * ----- | 1-15 | INV. A61N1/32 A61N1/36 |
| A | US 2018/185649 A1 (MICHAELI SHALOM [US] ET AL) 5 July 2018 (2018-07-05) * abstract * * paragraph [0041] - paragraph [0043] * * figures 7A-C * ----- | 1-15 | |
| A,D | WO 2016/057855 A1 (MASSACHUSETTS INST TECHNOLOGY [US]) 14 April 2016 (2016-04-14) * the whole document * ----- | 1-15 | |

TECHNICAL FIELDS
SEARCHED    (IPC)

A61N

The present search report has been drawn up for all claims

| Place of search | Date of completion of the search | Examiner |
|---|---|---|
| Munich | 21 June 2024 | Artikis, T |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

 

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 23 20 8090

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2024

| Patent document cited in search report | Publication date | Patent family member(s) | | Publication date |
|---|---|---|---|---|
| WO 2021044168 A1 | 11-03-2021 | EP | 4025294 A1 | 13-07-2022 |
| | | GB | 2591429 A | 04-08-2021 |
| | | US | 2022331602 A1 | 20-10-2022 |
| | | WO | 2021044168 A1 | 11-03-2021 |
| US 2018185649 A1 | 05-07-2018 | NONE | | |
| WO 2016057855 A1 | 14-04-2016 | DK | 3204113 T3 | 04-03-2024 |
| | | EP | 3204113 A1 | 16-08-2017 |
| | | FI | 3204113 T3 | 27-02-2024 |
| | | US | 2017216594 A1 | 03-08-2017 |
| | | US | 2019117975 A1 | 25-04-2019 |
| | | US | 2021121693 A1 | 29-04-2021 |
| | | WO | 2016057855 A1 | 14-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016057855 A1

- WO 2021044168 A1